# EUROPÄISCHE PATENTSCHRIFT

(11) **EP 1 358 484 B1**
(45) Veröffentlichungstag und Bekanntmachung des Hinweises auf die Patenterteilung: **07.10.2009**
(21) Anmeldenummer: 02712883.4
(22) Anmeldetag: 01.02.2002
(51) Int. Cl.: G01N 33/543, C12Q 1/68

(54) **VERFAHREN ZUR FÄLSCHUNGSSICHEREN MARKIERUNG; FÄLSCHUNGSSICHERE MARKIERUNG UND KIT**
METHOD FOR FORGERY-PROOF MARKING; FORGERY-PROOF MARKING AND KIT
PROCEDE DE MARQUAGE INFALSIFIABLE, MARQUAGE INFALSIFIABLE ET KIT

(30) Priorität: 05.02.2001 DE 10105339
(43) Veröffentlichungstag der Anmeldung: 05.11.2003
(73) Patentinhaber: November Aktiengesellschaft Gesellschaft für Molekulare Medizin, 91056 Erlangen (DE)
(72) Erfinder: BAUER, Georg, 90409 Nürnberg (DE); JOSTEN, André, 90429 Nürnberg (DE); WALTER, Harald, 8810 Horgen (CH); HASSMANN, Jörg, 91052 Erlangen (DE)
(74) Vertreter: UEXKÜLL & STOLBERG
(86) Internationale Anmeldenummer: PCT/EP2002/001058
(87) Internationale Veröffentlichungsnummer: WO 2002/072878

(56) Entgegenhaltungen:
- EP-A- 0 745 690
- WO-A-87/06383
- DE-A- 19 811 730
- US-A- 5 139 812
- US-A- 5 866 336

## Beschreibung

Die Erfindung betrifft ein Verfahren zur fälschungssicheren Markierung, eine fälschungssichere Markierung nach dem Oberbegriff des Anspruchs 19 und einen Kit mit einer fälschungssicheren Markierung.

Die Erfindung betrifft insbesondere den Sicherheits-, Kodierungs- und Identifikationsbereich. '

Aus der DE 197 38 816 A1 ist es bekannt, an einen Feststoff zur Markierung gebundene Nukleinsäuren zu verwenden. Zur Detektion müssen die Nukleinsäuren allerdings vom Feststoff durch ein Extraktionsverfahren entfernt werden. Die in Lösung vorliegenden Nukleinsäuren müssen dann mittels einer spezifischen Reaktion, wie der PCR, vervielfältigt werden. In nachfolgenden Schritten wird die vervielfältigte Nukleinsäuresequenz analysiert. Das Verfahren ist zeit- und arbeitsaufwendig und nicht für einen Nachweis der Echtheit vor Ort geeignet. Zudem ist eine Extraktion der zur Markierung aufgebrachten Nukleinsäuren nicht bei jedem Feststoff möglich oder erwünscht.

Ein weiteres Verfahren zum Identifizieren einer an einem Festkörper vorgesehenen Markierung ist aus der DE 198 11 730 A1 bekannt. Die Markierung weist als Sonde eine an eine feste Phase gebundene Nukleotidsequenz auf. Die Nukleotidsequenz wird mit einer korrespondierenden Nukleotidsequenz in Kontakt gebracht, die an eine weitere feste Phase eines Detektionsmittels gebunden ist. Dieses Verfahren ist nur für plane Oberflächen, die einen engen Kontakt von Markierungs- und Detektionsseite ermöglichen, geeignet. Das Binden der Sonde und des Detektionsmittels an feste Phasen ist aufwendig. Die an den festen Phasen gebundenen Markierungs- und Detektionsmoleküle sind gegen mechanische Beanspruchung instabil und gegenüber Verschmutzung anfällig, was eine geringe Stabilität der Markierung in sich birgt.

Aus der gattungsbildenden US 5,139,812 ist es bekannt, eine vorgegebene Nukleinsäure enthaltende Tinte zur fälschungssicheren Markierung von Gegenständen zu verwenden. Die Markierung ist an einer geheimen Stelle eines wertvollen Gegenstandes aufgebracht. Um eine Mehrzahl von Gegenständen unterscheidbar zu markieren, werden mit der Tinte unterschiedliche Beschriftungen aufgebracht. Die Identifikation einer solchermaßen aufgebrachten Markierung erfolgt durch Bindung einer weiteren Nukleinsäure an die vorgegebene Nukleinsäure, und durch Identifikation der Bindung. Dazu muß die Markierung vom Gegenstand entfernt und mittels eines mehrstufigen Verfahrens, z.B. mittels Antikörper oder durch die Identifizierung einer radioaktiven Markierung, nachgewiesen werden. Die Identifikation der Markierung ist aufwendig. Sie kann nicht vor Ort durchgeführt werden. Ein ähnliches Verfahren ist auch aus der WO 87/06383 bekannt.

Die EP 0 745 690 A2 beschreibt sogenannte "molecular beacons" und deren Anwendung für die Hybridisierung. Eine Verwendung zur Detektion von Markierungen ist aus diesem Dokument nicht bekannt.

Die US 5,866,336 beschreibt mit einem Fluorophor markierte Primer. Die Primer werden mittels der Polymerase-Kettenreaktion amplifiziert. Im hybridisierten Zustand wird eine Rückfaltung der Primer aufgelöst. Das Fluoreszenzverhalten des am Primer vorgesehenen Fluorophors ändert sich damit. Das bekannte Verfahren ist für eine schnelle Identifikation einer Markierung ungeeignet, weil es die kosten- und zeitaufwendige Polymerase-Kettenreaktion erfordert.

Die DE 199 01 761 offenbart ein Verfahren zum Nachweis der Hybridisierung von DNA mittels Änderung eines Redox-Potentials. Eine solche Änderung des Redox-Potentials ist nicht ohne weiteres zu erfassen. Das bekannte Verfahren erlaubt ebenfalls keine schnelle und einfache Identifikation einer Markierung.

Aufgabe der vorliegenden Erfindung ist es, die Nachteile nach dem Stand der Technik zu beseitigen. Insbesondere sollen ein Verfahren und eine fälschungssichere Markierung angegeben werden, die eine einfache und schnelle Identifizierung der Markierung vor Ort erlaubt.

Diese Aufgabe wird durch die Merkmale der Ansprüche 1, 19 und 33 gelöst. Die Ansprüche 2 bis 18, 20 bis 32 und 34 bis 37 geben weitere vorteilhafte Merkmale an.

Nach Maßgabe der Erfindung ist ein Verfahren zur Identifizierung einer auf einem Gegenstand vorgesehenen fälschungssicheren Markierung vorgesehen, welche eine einen kommunizierenden Porenraum aufweisende Trägerschicht mit einer darin aufgenommenen aus ersten Biomolekülen gebildeten Sonde aufweist, wobei die Trägerschicht eine erste die Sonde enthaltende Markierungsfläche und eine zweite die Sonde nicht enthaltende Referenzfläche aufweist mit folgenden Schritten:
aa) Imprägnieren der Trägerschicht mit einem Identifizierungsmittel, welches zumindest abschnittsweise zu den ersten Biomolekülen komplementär ausgebildete zweite Biomoleküle enthält und
bb) Identifizieren einer in der Trägerschicht zwischen den ersten und den zweiten Biomolekülen auftretenden Reaktion, wobei die Markierungsfläche beobachtet und ein davon ausgehendes Fluoreszenzsignal ausgewertet wird, und wobei eine Kontrolle durch Beobachtung der Referenzfläche durchgeführt wird.

Das vorgeschlagene Verfahren erlaubt eine fälschungssichere Markierung und eine schnelle und einfache Identifizierung der Markierung.

Indem die Trägerschicht eine die erste Sonde enthaltende Markierungsfläche und eine die zweite Sonde nicht enthaltende Referenzfläche aufweist, ist eine Kontrolle des gemessenen Fluoreszenzsignals möglich. Die Beobachtung der Referenzfläche ermöglicht eine Aussage über den Hintergrund der Messung. So kann mit hoher Zuverlässigkeit eine Identifizierung der Markierung erfolgen. Der Begriff "Referenzfläche" ist allgemein zu verstehen. Sofern es sich bei der Trägerschicht um einen Bestandteil des zu markierenden Gegenstandes selbst handelt, kann es sich bei der Referenzfläche auch um die Oberfläche des markierten Gegenstands handeln. Die Referenzfläche kann auch mit der Markierungsfläche identisch sein, wenn die Referenzfläche vor dem Imprägnieren der Trägerschicht mit dem Identifizierungsmittel beobachtet und eine Messung des Hintergrunds durchgeführt wird. Danach kann die Trägerschicht mit dem Identifizierungsmittel imprägniert werden und dann das davon ausgehende Fluoreszenzsignal gemessen und ausgewertet werden.

Zur Identifizierung werden die Markierungsfläche und die Referenzfläche beobachtet und insbesondere die Differenz der davon ausgehenden Floureszenzsignale ausgewertet. Die Auswertung kann automatisch mit einem geeigneten Handgerät erfolgen.

Die Beobachtung eines von der Markierungsfläche ausgehenden Fluoreszenzsignals ermöglicht eine Detektion einer zwischen dem ersten und dem zweiten Biomolekül auftretenden spezifische Reaktion in einer Stufe, insbesondere unter Weglassung eines Waschschrittes oder des Hinzufügens weiterer chemischer Reagenzien. Es ist insbesondere nicht erforderlich, die zur Markierung verwendeten ersten Biomoleküle von der Trägerschicht zu entfernen, danach zu amplifizieren und anschließend einen Nachweis durch Hinzufügen zweiter Biomoleküle zu führen. Ferner ist es nicht erforderlich, nach dem Aufbringen des Identifizierungsmittels einen Waschschritt oder dgl. durchzuführen. Auch ist es nicht erforderlich zur Identifizierung der Markierung die Trägerschicht vom markierten Gegenstand zu entfernen. - Die vorgeschlagene Markierung läßt sich einfach und kostengünstig herstellen. Sie eignet sich hervorragend zur Markierung von in zunehmendem Ausmaß gefälschten Markenprodukten, z.B. Zigaretten, Kleidung, Autoersatzteilen und dgl.. Mit der vorgeschlagenen Markierung ist es dem Hersteller der Markenprodukte möglich, die z.B. bei Großhändlern auf Lager befindlichen Waren stichprobenartig auf deren Authentizität zu prüfen.

Nach einer vorteilhaften Ausgestaltung wird die Trägerschicht beim Schritt lit. bb) mit Licht einer vorgegebenen Wellenlänge bestrahlt, und es wird eine die spezifische Bindung des ersten mit dem zweiten Biomolekül anzeigende Fluoreszenzreaktion beobachtet. Eine solche Nachweisreaktion läßt sich einfach vor Ort mittels eines geeigneten Handgeräts durchführen.

Die Trägerschicht kann aus einem lichtdurchlässigen oder einem reflektierenden Material hergestellt sein. Es kann sich dabei z.B. um ein Glasfaserflies handeln. Die Glasfasern können verspiegelt sein. Mit dieser Maßnahme kann erheblich die aus der Trägerschicht ausgekoppelte Lichtausbeute erhöht werden.

Die Trägerschicht wird zweckmäßigerweise aus einem der folgenden Materialien hergestellt: Zellulose, Nitrozellulose, Nylon, Polyacrylamidgel, poröses SiO2, Glasfaserflies.

Die Markierungsfläche kann mit einem die Sonde enthaltenden Gemisch unterschiedlicher Biomoleküle versehen sein. Das erhöht die Fälschungssicherheit der Markierung. Potentiellen Fälschern ist nicht bekannt, welches der in der Trägerschicht aufgenommenen Biomoleküle als Markierung benutzt wird. Außerdem lassen sich die Biomoleküle kaum analysieren bzw. identifizieren.

Die Sonde ist zweckmäßigerweise aus einem der folgenden Biopolymere gebildet: synthetische einzelsträngige Nukleinsäuren oder deren natürliche und/oder synthetische Analoga, Antigene, Proteine, wie Antikörper, Antikörperfragmente, Derivate von Antikörpern oder Antikörperfragmenten, Nukleinsäure-bindende Proteine, Rezeptoren, Liganden. Auch ähnlich wirkende Biomoleküle können selbstverständlich zur Herstellung der Sonde benutzt werden.

Nach einer weiteren Ausgestaltung kann die Sonde in einer vorgegebenen geometrischen Anordnung auf der Trägerschicht aufgebracht werden. Sie kann mittels eines Druckverfahrens, z.B. mittels eines Tintenstrahldruckkopfs oder mittels Siebdruck, auf die Trägerschicht aufgetragen werden. Bei der geometrischen Anordnung kann es sich um ein vorgegebenes Muster, z.B. einen Barcode handeln.

Ferner ist es von Vorteil, daß die Trägerschicht eine erste Auftragfläche aufweist, die mit der Markierungsfläche oder einer Mehrzahl von Markierungsflächen über einen ersten Weg oder erste Wege verbunden ist. Die erste Auftragfläche kann auch mit der Referenzfläche oder einer Mehrzahl von Referenzflächen über einen zweiten Weg oder zweite Wege verbunden sein. Es kann auch eine zweite und/oder weitere Auftragflächen vorgesehen sein, welche mit einer oder mehreren Markierungs- und/oder Referenzflächen verbunden sind. In den vorgenannten Fällen wird das Identifizierungsmittel entlang des ersten und/oder zweiten Wegs mittels Kapillarkräften von der/den Auftragfläche/n zur Markierungs- und/pder Referenzfläche transportiert. Die Trägerschicht ist zweckmäßigerweise zumindest abschnittsweise mit einer Schutzschicht überdeckt, welche transparent ausgebildet sein kann. Mit den vorgenannten Merkmalen ist es möglich, die Auftragfläche z.B. als Ausnehmung in der Schutzschicht auszubilden. Die von der Auftragfläche entfernt angeordnete Markierungs- und/oder Referenzfläche/n können in diesem Fall von der Schutzschicht abgedeckt und vor Kontaminationen geschützt sein. Das erhöht weiter die Zuverlässigkeit des vorgeschlagenen Verfahrens. Die transparente Ausbildung der Schutzschicht ermöglicht eine fluoreszenzoptische Identifizierung der Markierung.

Nach einem weiteren vorteilhaften Ausgestaltungsmerkmal ist die Trägerschicht mittels eines Klebstoffs oder durch Laminierung am zu markierenden Gegenstand befestigt. Die Trägerschicht kann an ihrer befestigungsseitigen Oberfläche mit einer, vorzugsweise eine abziehbare Schutzfolie aufweisenden, Klebefolie versehen sein. Die Trägerschicht kann also nach Art eines selbstklebenden Etiketts ausgebildet sein.

Nach einer weiteren Ausgestaltung kann dem Identifizierungsmittel ein dessen Verbreitung in der Trägerschicht anzeigender Farbstoff zugesetzt werden. Das ist insbesondere dann von Vorteil, wenn die Markierungs- und/oder Referenzfläche/n entfernt von der Auftragfläche angeordnet sind. In diesem Fall kann mittels des Farbstoffs kontrolliert werden, ob das Identifizierungsmittel mittels Kapillarkräften tatsächlich bis zur Markierungs- und/oder Referenzfläche transportiert worden ist. Die Kontrolle der Verbreitung des Identifizierungsmittels kann auch durch eine Leitfähigkeitsmessung erfolgen.

Das Identifizierungsmittel kann mittels einer Kapillare auf die auf der Trägerschicht ausgebildete Auftragfläche aufgegeben werden. In der Kapillare kann auch einfache Weise eine geeignete vorgegebene Menge des Identifizierungsmittels aufgenommen sein. Das Identifizierungsmittel kann aber auch in einem Stift oder einer Pipette aufgenommen sein.

Nach weiterer Maßgabe der Erfindung ist bei einer fälschungssicheren Markierung vorgesehen, daß die Trägerschicht an eine erste die Sonde enthaltende Markierungsfläche und eine zweite die Sonde nicht enthaltende Referenzfläche aufweist. - Eine solche Markierung kann einfach, schnell und mit hoher Zuverlässigkeit vor Ort mittels fluoreszenzoptischer Methoden identifiziert werden. Es ist nicht erforderlich, eine solche Markierung zur Identifizierung vom markierten Gegenstand entfernen und mittels aufwendiger naßchemischer Methoden zu behandeln. Wegen der vorteilhaften Ausgestaltungen der Markierung wird auf die vorangegangenen Ausführungen verwiesen, welche sinngemäß auch für die beanspruchte fälschungssichere Markierung zutreffen.

Nach weiterer Maßgabe der Erfindung ist ein Kit mit einer erfindungsgemäßen fälschungssicheren Markierung und einer ein zur Sonde korrespondierendes zweites Biomolekül enthaltendes Identifizierungsmittel vorgesehen.

Nach einer besonders vorteilhaften Ausgestaltung des Kits kann das Identifizierungsmittel in einer Kapillare aufgenommen sein. Die Kapillare kann z.B. wie eine Miene in einer stiftartigen Halterung aufgenommen sein.

Nachfolgend werden Ausführungsbeispiele der Erfindung anhand der Zeichnungen näher erläutert. Es zeigen:
- Fig. 1a: eine Draufsicht auf eine zweite fälschungssichere Markierung,
- Fig. 1b: eine Querschnittsansicht nach Fig. 1a,
- Fig. 2a: eine Draufsicht auf eine zweite fälschungssichere Markierung,
- Fig. 2b: eine Querschnittsansicht nach Fig. 2a,
- Fig. 3: eine Draufsicht auf eine dritte fälschungssichere Markierung,
- Fig. 4 ': die Signalstärke in Abhängigkeit unterschiedlicher DNA-Sequenzen,
- Fig. 5: die Reproduzierbarkeit des Fluoreszenzsignals,
- Fig. 6: die Reproduzierbarkeit der in den Träger eingebrachten Menge an Identifizierungsmittel,
- Fig. 7: eine schematische Querschnittsansicht einer fälschungssicheren Markierung sowie eines Identifizierungsmittels und
- Fig. 8a - d: den Verfahrensablauf in schematischen Querschnittsansichten.

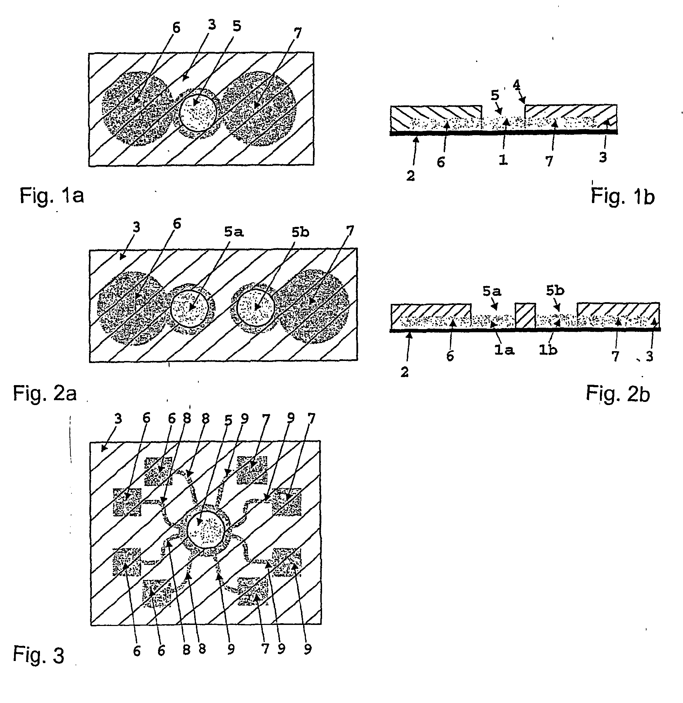

In den in Fig. 1a bis 3 sind verschiedene Ausführungsformen fälschungssicherer Markierungen gezeigt. Die fälschungssicheren Markierungen sind dabei jeweils nach Art eines Etiketts ausgeführt.

Bei der in den Fig. 1a und b gezeigten ersten fälschungssicheren Markierung ist mit dem Bezugszeichen 1 eine Trägerschicht bezeichnet, welche einen kommunizierenden Porenraum aufweist. Die Trägerschicht kann z.B. aus einem Filterpapier, einem Glasfaserfließ oder dgl. bestehen. In der Trägerschicht aufgenommen ist ein ersten Biomolekül, z.B. 3 pmol eines Oligonukleotids mit einer Länge von 30 bp. Das Biomolekül kann z.B. kovalent an die Trägerschicht gebunden sein. Die Trägerschicht 1 ist aufgebracht auf einen Träger 2. Dabei kann es sich um eine Kunststoff- oder Metallfolie oder einen Glasträger handeln, deren der Trägerschicht 1 abgewandte Seite mit einem druckempfindlichen Klebstoff beschichtet ist. Es ist aber auch möglich, die Trägerschicht 1 mittels eines doppelseitigen Klebebands auf dem Träger 2 zu befestigen. Die Deckschicht kann z.B. aus einer silikonisierten Kunststoffschicht oder einem silikonisierten Papier bestehen. Eine Schutzschicht 3 überdeckt randlich die Trägerschicht 1. Sie dient zur Fixierung der Trägerschicht 1 sowie zu deren Schutz. Eine in der Schutzschicht 3 vorgesehene Ausnehmung 4 begrenzt eine Auftragfläche 5. Die Auftragfläche 5 dient zur Aufnahme eines flüssigen Identifizierungsmittels. Das auf die Auftragfläche 5 aufgetragene flüssige Identifizierungsmittel wird mittels Kapillarkräften in das Innere der Trägerschicht 1 eingesaugt.

Bei der in den Fig. 1a und 1b gezeigten ersten fälschungssicheren Markierung ist die Trägerschicht 1 in Form dreier miteinander verbundener Kreisflächen ausgebildet. Eine erste Kreisfläche bildet eine Markierungsfläche 6, eine zweite damit verbundene Kreisfläche die Auftragfläche 5 und eine dritte mit der Auftragfläche 5 verbundene Kreisfläche eine Referenzfläche 7. Die so ausgebildete Trägerschicht 1 ist wiederum auf einem Träger 2 ausgebracht. Sie ist überdeckt mit einer, z.B. aus einer transparenten Kunststoffolie hergestellten, Schutzschicht 4. Die Schutzschicht 4 weist im Bereich der zweiten Kreisfläche eine kreisrunde Ausnehmung 4 auf, welche die Auftragfläche 5 bildet. Im vorliegenden Beispiel enthält lediglich die Markierungsfläche 6 das erste Biomolekül. Die Auftragfläche 5 und die Referenzfläche 7 enthalten nicht das erste Biomolekül. Im vorliegenden Ausführungsbeispiel sind die Markierungsfläche 6 und die Referenzfläche 7 vollflächig abgedeckt mit der Schutzschicht 3. Darin zur Identifizierung bzw. zur Referenz enthaltende Biomoleküle sind besonders gut geschützt. - Beim Aufbringen eines flüssigen Identifizierungsmittels auf die Auftragfläche 5 wird dieses mittels Kapillarkräften sowohl in die Markierungsfläche 6 als auch in die Referenzfläche 7 gesaugt. Es kommt dort ggf. zur Reaktion des ersten Biomoleküls mit einem im Identifizierungsmittel enthaltenen dazu korrespondierenden zweiten Biomolekül, welches z.B. als molecular beacon ausgebildet sein kann. Bei der Reaktion auftretendes Fluoreszenzlicht wird durch die transparente Schutzschicht 3 ausgekoppelt und kann als Identifizierungssignal beobachtet werden.

Bei der in den Fig. 2a und b gezeigten zweiten fälschungssicheren Markierung steht eine erste Auftragfläche 5a in Verbindung mit der Markierungsfläche 6. Eine zweite Auftragfläche 5b steht in Verbindung mit der Referenzfläche 7. Die erste Auftragfläche 5a und die Markierungsfläche 6 sind Bestandteil einer ersten Trägerschicht 1a, die zweite Auftragfläche 5b und die damit verbundene Referenzfläche 7 sind Bestandteil einer zweiten Trägerschicht 1b. Die erste Träger-, schicht 1a und zweite Trägerschicht 1b sind voneinander getrennt. Bei dieser Ausführungsform ist es möglich, die erste Auftragfläche 5a und zweite Auftragfläche 5b mit unterschiedlichen Identifizierungssubstanzen zu beaufschlagen.

Fig. 3 zeigt eine Draufsicht einer dritten fälschungssicheren Markierung. Die Auftragfläche 5 ist hier über erste Wege 8 mit einer Mehrzahl an Markierungsflächen verbunden. Sie ist ferner verbunden über zweite Wege 9 mit einer Mehrzahl an Referenzflächen 7. Ein auf die Auftragfläche 5 aufgebrachtes flüssiges Identifizierungsmittel wird mittels Kapillarkräften über die ersten 8 und die zweiten Wege 9 zu den Markierungs-6 und Referenzflächen 7 transportiert. Die Markierungs- 7 und Referenzflächen 8 sind jeweils vollflächig mit der Schutzschicht 3 überdeckt.

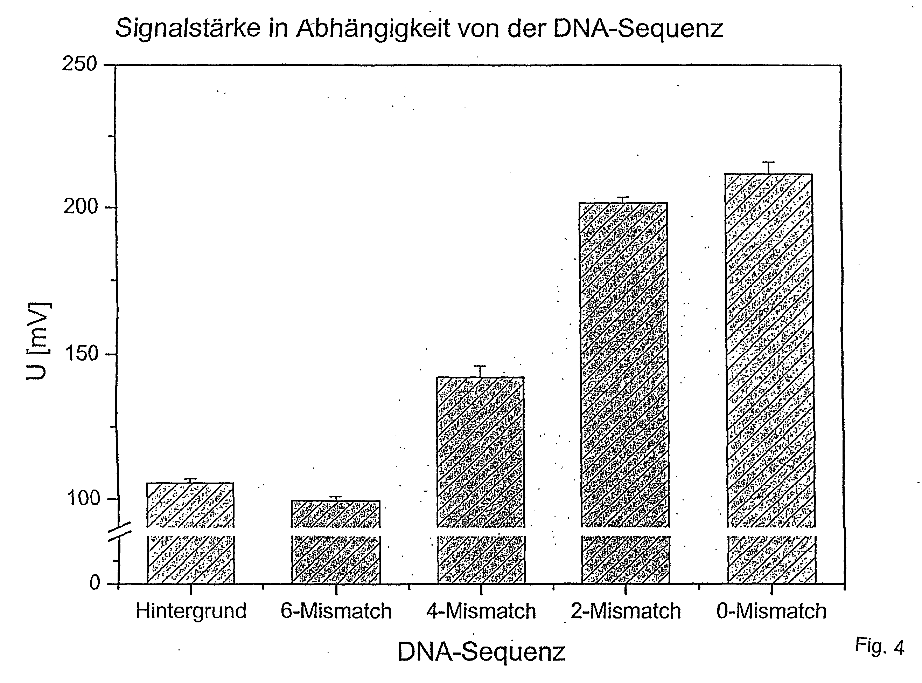

Fig. 4 zeigt im Vergleich die Stärke eines Fluoreszenzsignals, welche auf der Y-Achse in mV angegeben ist. Dargestellt sind die Ergebnisses des Hintergrunds, einer Hybridisierung mit einem moleculare beacon, wobei entweder 6, 4, 2 oder 0 Basenfehlpaarungen entlang des hybridisierten Abschnitts auftreten. Bereits eine Fehlpaarung von 2 Basen ist mit dem vorliegenden Verfahren unterscheidbar. Eine Fehlpaarung von 4 Basen führt zu einem drastisch niedrigeren Signal. Das belegt die hohe Spezifität des erfindungsgemäßen Verfahrens.

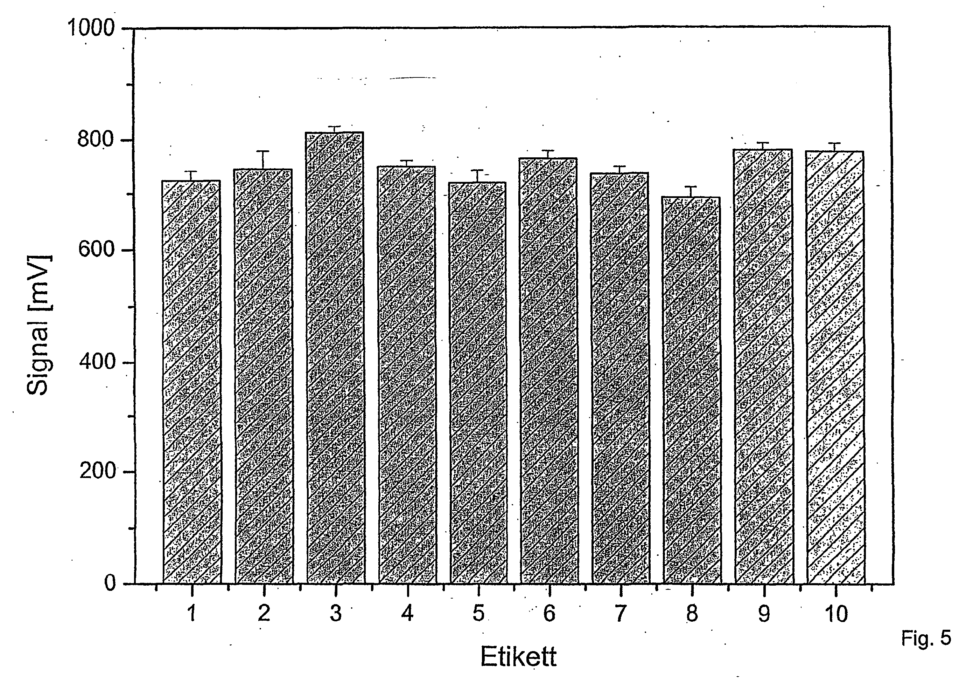

In Fig. 5 ist die Signalintensität auf der Y-Achse in mV wiedergegeben. Geprüft worden ist hier die Reproduzierbarkeit eines Signals bei wiederholter Verwendung eines Identifikationsmittels mit ein und demselben molecular beacon. Es zeigt sich, daß das auftretende Signal eine Schwankung von 4,7% gegenüber einem Mittelwert aufweist.

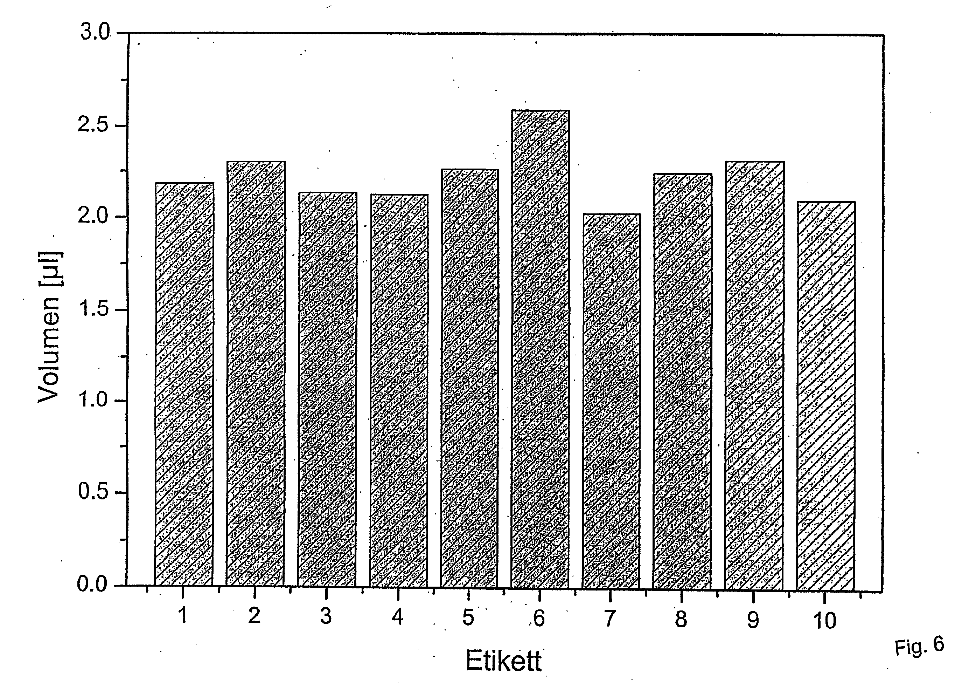

In Fig. 6 ist die Reproduzierbarkeit der Befüllung einer vorgegebenen Trägerschicht gezeigt. Auf der Y-Achse aufgetragen ist das in der Trägerschicht jeweils aufgenommene Volumen an Identifizierungsmittel. Der Befüllungsgrad ist gravimetrisch bestimmt worden. Er zeigt eine mittlere Abweichung von 7,2% gegenüber einem Mittelwert.

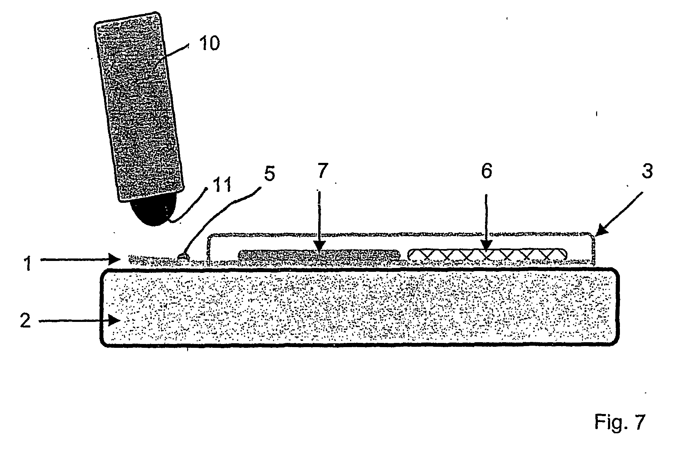

Fig. 7 zeigt eine schematische Querschnittsansicht eines Ausführungsbeispiels des Verfahrens. Dabei ist ein flüssiges Identifizierungsmittel in einer Kapillare 10 in einer Menge von 1 µl aufgenommen. Die Kapillare 10 kann z.B. nach Art einer Miene in einem Stift gehalten sein. Das Identifizierungsmittel enthält zweckmäßigerweise in einer Konzentration von 1 pmol/µl ein molecular beacon in einem Dig Easyhyb-Puffer (Roche, Biomedicals). Der Lösung kann ein gelber Farbstoff, z.B. der Lebensmittelfarbstoff E 104 zugesetzt sein. Das Identifizierungsmittel 11 kann aus der Kapillare 10 aufgetropft werden auf eine Auftragfläche 5 der Trägerschicht 1. Im Bereich der Auftragfläche 5 ist die Trägerschicht 1 nicht von der Schutzschicht 3 abgedeckt. Die Markierungsfläche 6 ist hier als eine einen kommunizierenden Porenraum aufweisende erste Schicht ausgebildet, welche auf der Trägerschicht 1 aufliegt. Die Referenzfläche 7 ist hier aus einer zweiten einen kommunizierenden Porenraum aufweisenden Schicht ausgebildet, welche ebenfalls auf der Trägerschicht aufliegt. Die erste und/oder zweite Schicht kann z.B. aus einer Nylon-Membran (Amersham Hybond N+) hergestellt sein, wobei darin 3 pmol eines 30 bp Oligonukleotids als erstes Biomolekül aufgenommen sind. Sowohl die Markierungsfläche 6 als auch die Referenzfläche 7 sind überdeckt von der Schutzschicht 3, welche als transparente Kunststoffolie ausgebildet ist.

Auf die Auftragfläche 5 aufgebrachtes Identifizierungsmittel wird mittels Kapillarkräften zur Markierungs- 6 und zur Referenzfläche 7 transportiert. Ein eventuell auftretendes Signal wird über die transparente Schutzschicht 3 ausgekoppelt.

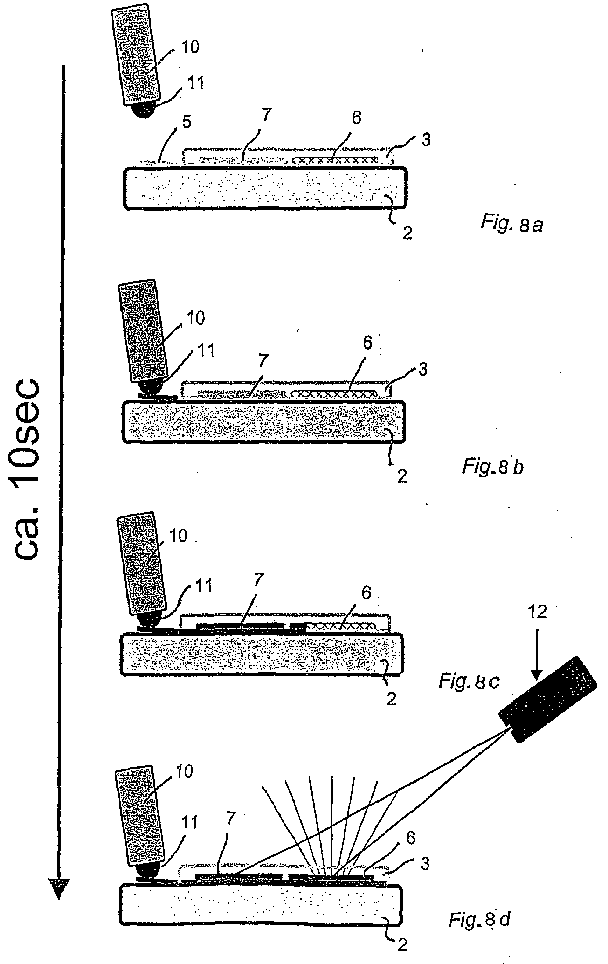

In den Fig. 8a bis d ist das erfindungsgemäße Verfahren nochmals in Einzelschritten schematisch gezeigt.

Das mittels Kapillarkräften in die Markierungs- 6 und Referenzfläche 7 eingesaugte Identifizierungsmittel wird mit einer Anregungslichtquelle 12 bestrahlt. Die in der Markierungsfläche 6 enthaltenen ersten Biomoleküle hybridisieren mit in der Identifizierungssubstanz 11 enthaltenen zweiten Biomolekülen, welche zumindest abschnittsweise korrespondierend zu den ersten Biomolekülen ausgebildet sind. Die zweiten Biomoleküle sind zweckmäßigerweise als molecular beacon ausgeführt. Das molecular beacon kann mit einem NIR-Fluorophor und einem dafür geeigneten Quencher am 3' bzw. 5' Ende versehen sein. Zweckmäßigerweise wird als Fluorophor Cy 5 (Amersham) und als Quencher BHQ 3 (Biosearch Technologies Inc.) verwendet. Bei der Hybridisierung kommt es zu einer Änderung der Sekundärstruktur des molecular beacons. Ein am molecular beacon vorgesehene fluorophore Markierung kann nach erfolgter Hybridisierung mittels einer Anregungslichtquelle 12, z.B. eine Laserdiode, angeregt werden. Das Anregungslicht kann mit einem herkömmlichen polymerischen Filter Roscolene 862 - True Blue - (Rosco) gefiltert werden. Das vom Fluorophor abgestrahlte Fluoreszenzlicht koppelt aus der Schutzschicht 3 aus und kann mittels einer Photodiode beobachtet werden. Das Auftreten des Fluoreszenzsignals zeigt die Authentizität der Markierung an.

Wie aus den Fig. 8a bis d ersichtlich ist, kann die Authensität der Markierung schnell und einfach vor Ort geprüft werden. Der Prüfvorgang nimmt lediglich etwa 10 Sekunden in Anspruch. Es ist kein Waschvorgang oder kein Entfernen der Markierung vom markierten Gegenstand erforderlich. Das vorgeschlagene Verfahren sowie die fälschungssichere Markierung eignet sich hervorragend zur Markierung von Massenprodukten. Deren Identifizierung kann mit einem kostengünstig herstellbaren Handgerät erfolgen.

Bezugszeichenliste
- 1: Trägerschicht
- 2: Träger
- 3: Schutzschicht
- 4: Ausnehmung
- 5: Auftragfläche
- 6: Markierungsfläche
- 7: Referenzfläche
- 8: erster Weg
- 9: zweiter Weg
- 10: Kapillare
- 11: Identifizierungssubstanz
- 12: Anregungslichtquelle

## Patentansprüche

1. Verfahren zur Identifizierung einer auf einem Gegenstand vorgesehenen fälschungssicheren Markierung, welche eine einen kommunizierenden Porenraum aufweisende Trägerschicht (1, 1a, 1b) mit einer darin aufgenommenen aus ersten Biomolekülen gebildeten Sonde aufweist,
wobei die Trägerschicht (1, 1a, 1b) eine erste die Sonde enthaltende Markierungsfläche (6) und eine zweite die Sonde nicht enthaltende Referenzfläche (7) aufweist,
mit folgenden Schritten:
aa) Imprägnieren der Trägerschicht (1, 1a, 1b) mit einem Identifizierungsmittel (11), welches zumindest abschnittsweise zu den ersten Biomolekülen komplementär ausgebildete zweite Biomoleküle enthält und
bb) Identifizieren einer in der Trägerschicht (1, 1a, 1b) zwischen den ersten und den zweiten Biomolekülen auftretenden Reaktion,
wobei die Markierungsfläche (6) beobachtet und ein davon ausgehendes Fluoreszenzsignal ausgewertet wird, und wobei eine Kontrolle durch Beobachtung der Referenzfläche durchgeführt wird.

2. Verfahren nach Anspruch 1, wobei die Trägerschicht (1, 1a, 1b) beim Schritt lit. bb mit Licht einer vorgegebenen Wellenlänge bestrahlt, und eine die spezifische Bindung des ersten mit dem zweiten Biomolekül anzeigende Fluoreszenzreaktion beobachtet wird.

3. Verfahren nach einem der vorhergehenden Ansprüche, wobei die Trägerschicht (1, 1a, 1b) aus einem lichtdurchlässigen oder einem reflektierenden Material hergestellt ist.

4. Verfahren nach einem der vorhergehenden Ansprüche, wobei die Trägerschicht (1, 1a, 1b) aus einem der folgenden Materialien hergestellt wird: Zellulose, Nitrozellulose, Nylon, Polyacrylamidgel, poröses SiO2, Glasfaserflies.

5. Verfahren nach einem der vorhergehenden Ansprüche, wobei die Markierungsfläche mit einem die Sonde enthaltenden Gemisch unterschiedlicher Biomoleküle versehen ist.

6. Verfahren nach einem der vorhergehenden Ansprüche, wobei die Sonde aus einem der folgenden Biopolymere gebildet ist: synthetische einzelsträngige Nukleinsäuren oder deren natürliche und/oder synthetische Analoga, Antigene, Proteine, wie Antikörper, Antikörperfragmente, Derivate von Antikörpern oder Antikörperfragmenten, Nukleinsäure-bindende Proteine, Rezeptoren, Liganden.

7. Verfahren nach einem der vorhergehenden Ansprüche, wobei die Sonde in einer vorgegebenen geometrischen Anordnung auf die Trägerschicht (1, 1a, 1b) aufgebracht wird.

8. Verfahren nach einem der vorhergehenden Ansprüche, wobei die Sonde mittels eines Druckverfahrens, vorzugsweise mittels eines Tintenstrahldruckkopfs, auf die Trägerschicht (1, 1a, 1b) aufgetragen wird.

9. Verfahren nach einem der vorhergehenden Ansprüche, wobei die Trägerschicht (1, 1a, 1b) eine erste Auftragfläche (5a) aufweist, die mit der Markierungsfläche (6) oder einer Mehrzahl von Markierungsflächen über einen ersten Weg (8) oder erste Wege verbunden ist.

10. Verfahren nach einem der vorhergehenden Ansprüche, wobei die erste Auftragfläche (5a) mit der Referenzfläche (7) oder einer Mehrzahl an Referenzflächen über einen zweiten Weg (9) oder zweite Wege verbunden ist.

11. Verfahren nach einem der vorhergehenden Ansprüche, wobei eine zweite und/oder weitere Auftragflächen (5, 5a, 5b) vorgesehen sind, welche mit einer oder mehreren Markierungs- (6) und/oder Referenzflächen (7) verbunden sind.

12. Verfahren nach einem der vorhergehenden Ansprüche, wobei das Identifizierungsmittel (11) entlang eines ersten und/oder zweiten Wegs mittels Kapillarkräften von der Auftragfläche (5, 5a, 5b) zur Markierungs- (6) und/oder Referenzfläche (7) transportiert wird.

13. Verfahren nach einem der vorhergehenden Ansprüche, wobei die Trägerschicht (1, 1a, 1b) zumindest abschnittsweise mit einer Schutzschicht (3) überdeckt ist.

14. Verfahren nach einem der vorhergehenden Ansprüche, wobei die Schutzschicht (3) transparent ausgebildet ist.

15. Verfahren nach einem der vorhergehenden Ansprüche, wobei die Trägerschicht (1, 1a, 1b) mittels eines Klebstoffs oder durch Laminierung am zu markierenden Gegenstand befestigt wird.

16. Verfahren nach einem der vorhergehenden Ansprüche, wobei die Trägerschicht (1, 1a, 1b) an ihrer befestigungsseitigen Oberfläche mit einer, vorzugsweise eine abziehbare Schutzfolie aufweisenden, Klebefolie versehen ist.

17. Verfahren nach einem der vorhergehenden Ansprüche, wobei dem Identifizierungsmittel (11) ein dessen Verbreitung in der Trägerschicht (1, 1a, 1b) anzeigender Farbstoff zugesetzt wird.

18. Verfahren nach einem der vorhergehenden Ansprüche, wobei das Identifizierungsmittel (11) mittels einer Kapillare (10) eines Stifts oder einer Pipette auf die auf der Trägerschicht (1, 1a, 1b) ausgebildete Auftragfläche (5, 5a, 5b) aufgegeben wird.

19. Fälschungssichere Markierung mit einer einen kommunizierenden Porenraum aufweisenden Trägerschicht (1, 1a, 1b) und einer darin aufgenommenen aus einem ersten Biomolekül gebildeten Sonde,
**dadurch gekennzeichnet, daß**
die Trägerschicht (1, 1a, 1b) eine erste die Sonde enthaltende Markierungsfläche (6) und eine zweite die Sonde nicht enthaltende Referenzfläche (7) aufweist.

20. Fälschungssichere Markierung nach Anspruch 19, wobei die Trägerschicht (1, 1a, 1b) aus einem lichtdurchlässigen oder einem reflektierenden Material hergestellt ist.

21. Fälschungssichere Markierung nach einem der Ansprüche 19 oder 20, wobei die Trägerschicht (1, 1a, 1b) aus einem der folgenden Materialien hergestellt ist: Zellulose, Nitrozellulose, Nylon, Polyacrylamidgel, poröses SiO2, Glasfaserflies.

22. Fälschungssichere Markierung nach einem der Ansprüche 19 bis 21, wobei die Markierung aus einem die Sonde enthaltenden Gemisch unterschiedlicher Biomoleküle gebildet ist.

23. Fälschungssichere Markierung nach einem der Ansprüche 19 bis 22, wobei die Sonde aus einem der folgenden Biopolymere gebildet ist: synthetische einzelsträngige Nukleinsäuren oder deren natürliche und/oder synthetische Analoga, Antigene, Proteine, wie Antikörper, Antikörperfragmente, Derivate von Antikörpern oder Antikörperfragmenten, Nukleinsäure-bindende Proteine, Rezeptoren, Liganden.

24. Fälschungssichere Markierung nach einem der Ansprüche 19 bis 23, wobei die Sonde in einer vorgegebenen geometrischen Anordnung auf der Trägerschicht (1, 1a, 1b) aufgebracht ist.

25. Fälschungssichere Markierung nach einem der Ansprüche 19 bis 24, wobei die Sonde mittels eines Druckverfahrens, vorzugsweise mittels eines Tintenstrahldruckkopfs, auf die Trägerschicht (1, 1a, 1b) aufgebracht ist.

26. Fälschungssichere Markierung nach einem der Ansprüche 19 bis 25, wobei die Trägerschicht (1, 1a, 1b) eine erste Auftragfläche (5a) aufweist, die mit der Markierungsfläche (6) oder einer Mehrzahl von Markierungsflächen über einen ersten Weg (8) oder erste Wege verbunden ist.

27. Fälschungssichere Markierung nach einem der Ansprüche 19 bis 26, wobei die erste Auftragfläche (5a) mit der Referenzfläche (7) oder einer Mehrzahl an Referenzflächen über einen zweiten Weg (9) oder zweite Wege verbunden ist.

28. Fälschungssichere Markierung nach einem der Ansprüche 19 bis 27, wobei eine zweite und/oder weitere Auftragflächen (5, 5a, 5b) vorgesehen sind, welche mit einer oder mehreren Markierungs- (6) und/oder Referenzflächen (7) verbunden sind.

29. Fälschungssichere Markierung nach einem der Ansprüche 19 bis 28, wobei die Trägerschicht (1, 1a, 1b) zumindest abschnittsweise mit einer Schutzschicht (3) überdeckt ist.

30. Fälschungssichere Markierung nach einem der Ansprüche 19 bis 29, wobei die Schutzschicht (3) transparent ausgebildet ist.

31. Fälschungssichere Markierung nach einem der Ansprüche 19 bis 30, wobei die Trägerschicht (1, 1a, 1b) mittels eines Klebstoffs oder einer Laminierung am zu markierenden Gegenstand befestigt ist.

32. Fälschungssichere Markierung nach einem der Ansprüche 19 bis 31, wobei die Trägerschicht (1, 1a, 1b) an seiner befestigungsseitigen Oberfläche mit einer, vorzugsweise eine abziehbare Schutzfolie aufweisenden, Klebefolie versehen ist.

33. Kit mit einer fälschungssicheren Markierung nach einem der Ansprüche 19 bis 32 und einem zur Sonde korrespondierenden zweite Biomoleküle enthaltenden Identifizierungsmittel (11).

34. kit nach Anspruch 33, wobei die Sonde aus einem der folgenden Biopolymere gebildet ist: synthetische einzelsträngige Nukleinsäuren oder deren natürliche und/oder synthetische Analoga, Antigene, Proteine, wie Antikörper, Antikörperfragmente, Derivate von Antikörpern oder Antikörperfragmenten, Nukleinsäure-bindende Proteine, Rezeptoren, Liganden.

35. Kit nach Anspruch 34, wobei das Identifizierungsmittel ein zumindest abschnittsweise komplementär zum ersten Biopolymer ausgebildetes molecular beacon ist.

36. Kit nach einem der Ansprüche 33 bis 35, wobei das Identifizierungsmittel (11) in einer Kapillare (10), einem Stift oder einer Pipette aufgenommen ist.

37. Kit nach einem der Ansprüche 33 bis 36, wobei dem Identifizierungsmittel (11) ein Farbstoff beigemischt ist.

## Claims

1. Method for the identification of a forgery-proof marking which is provided on an object that has a carrier layer (1, 1a, 1b) having a communicating pore chamber with a probe accommodated therein and formed from first biomolecules,
the carrier layer (1, 1a, 1b) having a first, marking area (6) containing the probe and a second, reference area (7) not containing the probe,
comprising the following steps:
aa) impregnating the carrier layer (1, 1a, 1b) with an identification means (11) which, at least in some sections, contains second biomolecules that are formed so as to be complementary to the first biomolecules, and
bb) identifying a reaction occurring between the first and the second biomolecules in the carrier layer (1, 1a, 1b),
the marking area (6) being observed and a fluorescence signal originating therefrom being evaluated, and an inspection being carried out by observing the reference area.

2. Method according to Claim 1, the carrier layer (1, 1a, 1b) in step letter bb being irradiated with light of a predefined wavelength, and a fluorescent reaction indicating the specific binding of the first with the second biomolecule being observed.

3. Method according to one of the preceding claims, the carrier layer (1, 1a, 1 b) being produced from a translucent or a reflective material.

4. Method according to one of the preceding claims, the carrier layer (1, 1a, 1b) being produced from one of the following materials: cellulose, nitrocellulose, nylon, polyacrylamide gel, porous SiO₂, glass-fibre nonwoven.

5. Method according to one of the preceding claims, the marking area being provided with a mixture of different biomolecules containing the probe.

6. Method according to one of the preceding claims, the probe being formed from one of the following biopolymers: synthetic single-strand nucleic acids or their natural and/or synthetic analogues, antigens, proteins, such as antibodies, antibody fragments, derivatives of antibodies or antibody fragments, nucleic acid-binding proteins, receptors, ligands.

7. Method according to one of the preceding claims, the probe being applied to the carrier layer (1, 1a, 1 b) in a predefined geometric arrangement.

8. Method according to one of the preceding claims, the probe being applied to the carrier layer (1, 1a, 1 b) by means of a printing process, preferably by means of an ink jet print head.

9. Method according to one of the preceding claims, the carrier layer (1, 1 a, 1b) having a first application area (5a), which is connected to the marking area or a plurality of marking areas via a first path (8) or first paths.

10. Method according to one of the preceding claims, the first application- area (5a) being connected to the reference area (7) or a plurality of reference areas via a second path (9) or second paths.

11. Method according to one of the preceding claims, a second and/or further application areas (5, 5a, 5b) being provided, which are connected to one or more marking areas (6) and/or reference areas (7).

12. Method according to one of the preceding claims, the identification means (11) being transported from the application area (5, 5a, 5b) to the marking area (6) and/or reference area (7) along a first and/or second path by means of capillary forces.

13. Method according to one of the preceding claims, at least some sections of the carrier layer (1, 1a, 1 b) being covered with a protective layer (3).

14. Method according to one of the preceding claims, the protective layer (3) being formed so as to be transparent.

15. Method according to one of the preceding claims, the carrier layer (1, 1a, 1 b) being fixed to the object to be marked by means of an adhesive or by means of lamination.

16. Method according to one of the preceding claims, the carrier layer (1, 1 a, 1b) being provided on its surface on the fixing side with an adhesive film, preferably having a pull-off protective film.

17. Method according to one of the preceding claims, the identification means (11) having added to it a dyestuff indicating its propagation in the carrier layer (1, 1 a, 1b).

18. Method according to one of the preceding claims, the identification means (11) being discharged onto the application area (5, 5a, 5b) formed on the carrier layer (1, 1 a, 1 b) by means of a capillary (10), a pen or a pipettes.

19. Forgery-proof marking having a carrier layer (1, 1a, 1b) having a communicating pore chamber and a probe accommodated therein and formed from a first biomolecule,
**characterized in that**
the carrier layer (1, 1a, 1b) has a first, marking area (6) containing the probe and a second, reference area (7) not containing the probe.

20. Forgery-proof marking according to Claim 19, the carrier layer (1, 1a, 1b) being produced from a translucent or a reflective material.

21. Forgery-proof marking according to either of Claims 19 and 20, the carrier layer (1, 1a, 1b) being produced from one of the following materials: cellulose, nitrocellulose, nylon, polyacrylamide gel, porous SiO₂, glass-fibre nonwoven.

22. Forgery-proof marking according to one of Claims 19 to 21, the marking being formed from a mixture of different biomolecules containing the probe.

23. Forgery-proof marking according to one of Claims 19 to 22, the probe being formed from one of the following biopolymers: synthetic single-strand nucleic acids or their natural and/or synthetic analogues, antigens, proteins, such as antibodies, antibody fragments, derivatives of antibodies or antibody fragments, nucleic acid-binding proteins, receptors, ligands.

24. Forgery-proof marking according to one of Claims 19 to 23, the probe being applied to the carrier layer (1, 1a, 1b) in a predefined geometric arrangement.

25. Forgery-proof marking according to one of Claims 19 to 24, the probe being applied to the carrier layer (1, 1a, 1b) by means of a printing process, preferably by means of an ink jet print head.

26. Forgery-proof marking according to one of Claims 19 to 25, the carrier layer (1, 1a, 1b) having a first application area (5a) which is connected to the marking area (6) or a plurality of marking areas via a first path (8) or first paths.

27. Forgery-proof marking according to one of Claims 19 to 26, the first application area (5a) being connected to the reference area (7) or a plurality of reference areas via a second path (9) or second paths.

28. Forgery-proof marking according to one of Claims 19 to 27, a second and/or further application areas (5, 5a, 5b) being provided, which are connected to one or more marking areas (6) and/or reference areas (7).

29. Forgery-proof marking according to one of Claims 19 to 28, at least some sections of the carrier layer (1, 1a, 1b) being covered with a protective layer (3).

30. Forgery-proof marking according to one of Claims 19 to 29, the protective layer (3) being formed so as to be transparent.

31. Forgery-proof marking according to one of Claims 19 to 30, the carrier layer (1, 1 a, 1b) being fixed to the object to be marked by means of an adhesive or a lamination.

32. Forgery-proof marking according to one of Claims 19 to 31, the carrier layer (1, 1 a, 1b) being provided on its surface on the fixing side with an adhesive film, preferably having a pull-off protective film.

33. Kit having a forgery-proof marking according to one of Claims 19 to 32 and an identification means (11) containing second biomolecules corresponding to the probe.

34. Kit according to Claim 33, the probe being formed from one of the following biopolymers: synthetic single-strand nucleic acids or their natural and/or synthetic analogues, antigens, proteins, such as antibodies, antibody fragments, derivatives of antibodies or antibody fragments, nucleic acid-binding proteins, receptors, ligands.

35. Kit according to Claim 34, the identification means being a molecular beacon formed, at least in some sections, in a manner complementary to the first biopolymer.

36. Kit according to one of Claims 33 to 35, the identification means (11) being accommodated in a capillary (10), a pen or a pipette.

37. Kit according to one of Claims 33 to 36, a dyestuff being mixed with the identification means (11).

## Revendications

1. Procédé d'identification d'un marquage infalsifiable prévu sur un objet et présentant une couche de support (1, 1a, 1b) ayant un espace cellulaire communiquant, avec une sonde reçue dans celui-ci et composée de premières biomolécules,
dans lequel la couche de support (1, 1a, 1b) présente une première surface de marquage (6) contenant la sonde et une deuxième couche de référence (7) ne contenant pas la sonde,
le procédé comprenant les étapes consistant à :
aa) imprégner la couche de support (1, 1a, 1b) d'un agent d'identification (11) qui contient au moins par sections des deuxièmes biomolécules réalisées de façon complémentaire aux premières biomolécules, et
bb) identifier une réaction se produisant entre les premières et les deuxièmes biomolécules dans la couche de support (1, 1a, 1b),
dans lequel la surface de marquage (6) est observée et un signal de fluorescence émanant de celle-ci est évalué, et dans lequel un contrôle est effectué par l'observation de la surface de référence.

2. Procédé selon la revendication 1, dans lequel la couche de support (1, 1a, 1b) à l'étape bb) est exposée à une lumière d'une longueur d'onde prédéfinie, et une réaction de fluorescence indiquant la liaison spécifique de la première avec la deuxième biomolécule est observée.

3. Procédé selon l'une quelconque des revendications précédentes, dans lequel la couche de support (1, 1a, 1b) est fabriquée à partir d'un matériau transparent ou réfléchissant.

4. Procédé selon l'une quelconque des revendications précédentes, dans lequel la couche de support (1, 1a, 1b) est fabriquée à partir d'un des matériaux suivants : de la cellulose, de la nitrocellulose, du nylon, un gel de polyacrylamide, un SiO₂ poreux, un voile de fibre de verre.

5. Procédé selon l'une quelconque des revendications précédentes, dans lequel la surface de marquage est munie d'un mélange de différentes biomolécules contenant la sonde.

6. Procédé selon l'une quelconque des revendications précédentes, dans lequel la sonde est formée à partir d'un des biopolymères suivants : des acides nucléiques synthétiques à chaîne unique ou leurs analogues naturels et/ou synthétiques, des antigènes, des protéines, comme des anticorps, des fragments d'anticorps, des dérivés d'anticorps ou de fragments d'anticorps, des protéines liant l'acide nucléique, des récepteurs, des ligands.

7. Procédé selon l'une quelconque des revendications précédentes, dans lequel la sonde est appliquée à la couche de support (1, 1a, 1b) dans une disposition géométrique prédéfinie.

8. Procédé selon l'une quelconque des revendications précédentes, dans lequel la sonde est appliquée à la couche de support (1, 1a, 1b) au moyen d'un procédé d'impression, de préférence au moyen d'une tête d'impression à jet d'encre.

9. Procédé selon l'une quelconque des revendications précédentes, dans lequel la couche de support (1, 1a, 1b) présente une première surface d'application (5a) qui est reliée à la surface de marquage (6) ou à une pluralité de surfaces de marquage par l'intermédiaire d'un premier trajet (8) ou de premiers trajets.

10. Procédé selon l'une quelconque des revendications précédentes, dans lequel la première surface d'application (5a) est reliée à la surface de référence (7) ou à une pluralité de surfaces de référence par l'intermédiaire d'un deuxième trajet (9) ou de deuxièmes trajets.

11. Procédé selon l'une quelconque des revendications précédentes, dans lequel une deuxième et/ou d'autres surfaces d'application (5, 5a, 5b) sont prévues qui sont reliées à une ou plusieurs surfaces de marquage (6) et/ou de référence (7).

12. Procédé selon l'une quelconque des revendications précédentes, dans lequel l'agent d'identification (11) est transporté suivant un premier et/ou deuxième trajet au moyen de forces capillaires de la surface d'application (5, 5a, 5b) à la surface de marquage (6) et/ou de référence (7).

13. Procédé selon l'une quelconque des revendications précédentes, dans lequel la couche de support (1, 1a, 1b) est recouverte au moins par sections d'une couche de protection (3).

14. Procédé selon l'une quelconque des revendications précédentes, dans lequel la couche de protection (3) est réalisée de façon transparente.

15. Procédé selon l'une quelconque des revendications précédentes, dans lequel la couche de support (1, 1a, 1b) est fixée à l'objet à marquer au moyen d'un adhésif ou par laminage.

16. Procédé selon l'une quelconque des revendications précédentes, dans lequel la couche de support (1, 1a, 1b) est munie sur sa surface côté fixation d'un film adhésif présentant de préférence un film de protection pelable.

17. Procédé selon l'une quelconque des revendications précédentes, dans lequel l'agent d'identification (11) est additionné d'un colorant indiquant la propagation de celui-ci dans la couche de support (1, 1a, 1b).

18. Procédé selon l'une quelconque des revendications précédentes, dans lequel l'agent d'identification (11) est appliqué à la surface d'application (5, 5a, 5b) réalisée sur la couche de support (1, 1a, 1b) au moyen d'un tube capillaire (10) d'un stylet ou d'une pipette.

19. Marquage infalsifiable avec une couche de support (1, 1a, 1b) présentant un espace cellulaire communiquant et avec une sonde reçue dans celui-ci et composée d'une première biomolécule,
**caractérisé en ce que**
la couche de support (1, 1a, 1b) présente une première surface de marquage (6) contenant la sonde et une deuxième couche de référence (7) ne contenant pas la sonde.

20. Marquage infalsifiable selon la revendication 19, dans lequel la couche de support (1, 1a, 1b) est fabriquée à partir d'un matériau transparent ou réfléchissant.

21. Marquage infalsifiable selon l'une quelconque des revendications 19 ou 20, dans lequel la couche de support (1, 1a, 1b) est fabriquée à partir d'un des matériaux suivants : de la cellulose, de la nitrocellulose, du nylon, un gel de polyacrylamide, un SiO₂ poreux, un voile de fibre de verre.

22. Marquage infalsifiable selon l'une quelconque des revendications 19 à 21, dans lequel le marquage est formé à partir d'un mélange de différentes biomolécules contenant la sonde.

23. Marquage infalsifiable selon l'une quelconque des revendications 19 à 22, dans lequel la sonde est formée à partir d'un des biopolymères suivants : des acides nucléiques synthétiques à chaîne unique ou leurs analogues naturels et/ou synthétiques, des antigènes, des protéines, comme des anticorps, des fragments d'anticorps, des dérivés d'anticorps ou de fragments d'anticorps, des protéines liant l'acide nucléique, des récepteurs, des ligands.

24. Marquage infalsifiable selon l'une quelconque des revendications 19 à 23, dans lequel la sonde est appliquée à la couche de support (1, 1a, 1b) dans une disposition géométrique prédéfinie.

25. Marquage infalsifiable selon l'une quelconque des revendications 19 à 24, dans lequel la sonde est appliquée à la couche de support (1, 1a, 1b) au moyen d'un procédé d'impression, de préférence au moyen d'une tête d'impression à jet d'encre.

26. Marquage infalsifiable selon l'une quelconque des revendications 19 à 25, dans lequel la couche de support (1, 1a, 1b) présente une première surface d'application (5a) qui est reliée à la surface de marquage (6) ou à une pluralité de surfaces de marquage par l'intermédiaire d'un premier trajet (8) ou de premiers trajets.

27. Marquage infalsifiable selon l'une quelconque des revendications 19 à 26, dans lequel la première surface d'application (5a) est reliée à la surface de référence (7) ou à une pluralité de surfaces de référence par l'intermédiaire d'un deuxième trajet (9) ou de deuxièmes trajets.

28. Marquage infalsifiable selon l'une quelconque des revendications 19 à 27, dans lequel une deuxième et/ou d'autres surfaces d'application (5, 5a, 5b) sont prévues qui sont reliées à une ou plusieurs surfaces de marquage (6) et/ou de référence (7).

29. Marquage infalsifiable selon l'une quelconque des revendications 19 à 28, dans lequel la couche de support (1, 1a, 1b) est recouverte au moins par sections d'une couche de protection (3).

30. Marquage infalsifiable selon l'une quelconque des revendications 19 à 29, dans lequel la couche de protection (3) est réalisée de façon transparente.

31. Marquage infalsifiable selon l'une quelconque des revendications 19 à 30, dans lequel la couche de support (1, 1a, 1b) est fixée à l'objet à marquer au moyen d'un adhésif ou par laminage.

32. Marquage infalsifiable selon l'une quelconque des revendications 19 à 31, dans lequel la couche de support (1, 1a, 1b) est munie sur sa surface côté fixation d'un film adhésif présentant de préférence un film de protection pelable.

33. Kit avec un marquage infalsifiable selon l'une quelconque des revendications 19 à 32 et un agent d'identification (11) correspondant à la sonde et contenant des deuxièmes biomolécules.

34. Kit selon la revendication 33, dans lequel la sonde est formée à partir d'un des biopolymères suivants : des acides nucléiques synthétiques à chaîne unique ou leurs analogues naturels et/ou synthétiques, des antigènes, des protéines, comme des anticorps, des fragments d'anticorps, des dérivés d'anticorps ou de fragments d'anticorps, des protéines liant l'acide nucléique, des récepteurs, des ligands.

35. Kit selon la revendication 34, dans lequel l'agent d'identification est un phare moléculaire réalisé au moins par sections de façon complémentaire au premier biopolymère.

36. Kit selon l'une quelconque des revendications 33 à 35, dans lequel l'agent d'identification (11) est reçu dans un tube capillaire (10), un stylet ou une pipette.

37. Kit selon l'une quelconque des revendications 33 à 36, dans lequel l'agent d'identification (11) est additionné d'un colorant.
